# EUROPEAN PATENT SPECIFICATION

(11) **EP 1 765 146 B1**
(45) Date of publication and mention of the grant of the patent: **29.04.2009**
(21) Application number: 05745079.3
(22) Date of filing: 30.05.2005
(51) Int. Cl.: A61B 1/05, A61B 1/00, G02B 23/24, H04N 5/225, A61B 1/04

(54) **A REUSABLE MINIATURE CAMERA HEAD**
WIEDERVERWENDBARER MINIATUR-KAMERAKOPF
TETE DE CAMERA MINIATURE REUTILISABLE

(30) Priority: 31.05.2004 IL 16225104
(43) Date of publication of application: 28.03.2007
(73) Proprietor: Medigus Ltd., 84965 Omer (IL)
(72) Inventor: SONNENSCHEIN, Elazar, 84800 Beer Sheva (IL); SONNENSCHEIN, Minelu, 85025 Meitar (IL); GOVRIN, Amir, 62336 Tel Aviv (IL); Sheinberg, Shai, 84816 Beer Sheva (IL)
(74) Representative: Tiesmeyer, Johannes
(86) International application number: PCT/IL2005/000555
(87) International publication number: WO 2005/115221

(56) References cited:
- EP-A- 0 790 652
- WO-A-97/24764
- WO-A-99/62121
- WO-A-02/087426
- WO-A-20/05002210
- FR-A- 2 737 650
- US-A- 4 888 639
- US-A- 5 313 306
- US-A- 5 913 817
- US-A- 6 095 970
- US-A1- 2002 128 535
- US-A1- 2004 061 776
- US-A1- 2004 077 121

## Description

### Field of the Invention

The present invention relates to the field of remote imaging. Particularly the invention relates to a reusable miniature camera head that can be attached to and detached from an object.

### Background of the Invention

Minimal access diagnostics and/or therapy, e.g. laparoscopy, interventional flexible endoscopy, minimal access surgery, and percutaneous interventional radiology, are generally carried out within the body cavities or small incisions in the abdomen and therefore the person carrying out the procedure cannot directly view the operating field. For this reason, the ability to carry out such procedures is dependent on the imaging systems that display the images obtained by the camera sensor focused on the scene.

The imaging systems comprise a miniature camera head based on solid-state sensors, e.g. Charge Coupled Device (CCD) and permanently attached to the distal tip of the rigid, semi-rigid, or flexible endoscope and connected, usually by means of a cable that runs through the length of the endoscope, to a power supply, computing device for processing the signals from the CCD, and display means.

The high cost of rigid, semi-rigid, and flexible endoscopes dictates that they must be reused for numerous procedures. This in turn dictates that they must be sterilized between each procedure, which further increases the expense, since they must be built using special techniques and special materials to be able to withstand the harsh chemicals and/or high temperatures and/or washing machines of the sterilization and cleaning procedure.

The camera assembly is by far the most expensive part of an endoscope and a significant reduction in the cost of the endoscopes and the procedures carried out using them could be achieved, if a way could be found to separate the camera head from the device after each use, discard the rest of the device, sterilize or clean the camera head only, and then attach it to a new rigid, semi-rigid, or flexible endoscope for the next procedure.

In co-pending International Patent Application WO2005/002210 by the same applicant, the description of which, including reference cited therein, is incorporated herein by reference in its entirety, there are described methods for producing an imager assembly for a miniature camera head. As the size of the CCD sensor on which the camera is based becomes smaller, a number of technical and practical problems have become apparent to the inventors. These problems are centered round the fact that the motivation behind using ever smaller sensors is to be able to produce smaller diameter endoscopes, which will be able to enter smaller diameter lumens in order to enable new therapeutic and diagnostic techniques. When the size of the CCD sensor becomes smaller than that disclosed in the above mentioned patent application, the size of the components of the electronic driver of the camera becomes the limiting factor that has to be overcome. Additionally, from a practical point-of-view, the detachable camera heads become so small that it is difficult to handle them.

US 6,095,970 discloses an endoscope with a detachable unit. US 2004/006 1776 A1 discloses a circuit substrate for forming a buffer amplifier located on a back surface of a CCD. WO 99/62121 A1 discloses glues to couple a sensor to a transceiver IC.

It is a purpose of the present invention to provide a miniature camera head that is smaller than any camera head that is presently available.

It is another purpose of the present invention to provide a detachable miniature camera head that can be attached to and detached from a rigid, semi-rigid, or flexible endoscope.

It is an additional purpose of the present invention to provide solutions to the problems that have arisen in trying to reduce the diameter of rigid, semi-rigid, and flexible endoscopes.

Further purposes and advantages of this invention will appear as the description proceeds.

### Summary of the Invention

The present invention is a miniature camera head according to claim 1.

The camera head of the invention can be based on the use of any type of semiconductor detector, e.g. a Charge Coupled Device (CCD); an Intensified Charge Coupled Device (ICCD); an Electron Multiplying Charge Coupled Device (EMCCD); or a Complementary Metal Oxide Semiconductor (CMOS) device, that is sensitive to radiation in any radiation band, e.g., X-ray, visible, or Near Infra Red (NIR). In preferred embodiments of the camera head of the invention the solid-state sensor is a Charge Coupled Device (CCD). In some embodiments at least some of the components of the electronic driver are located outside of the housing.

In typical applications, the object to which the camera head of the invention is attached is a rigid, semi-rigid, or flexible endoscope or catheter.

Henceforth in this application whenever the word endoscope is used it is to be understood in the broadest sense as including all types of rigid, semi-rigid, and flexible endoscopes, including laparoscopes with and without articulation sections, borescopes, catheters, etc.

In preferred embodiments of the invention, the camera head can be sterilized either chemically or in an autoclave. In other preferred embodiments of the invention, the camera head is a permanent part of a sterilizable distal tip, which can be detached and reattached to the insertion tube of an endoscopic or laparoscopic device.

The housing can have metallic electricity conducting strips arranged on its outside surface and the external electrical connector has metallic contacts arranged in such a way that they will engage the conducting strips when the housing is pushed into contact with the electrical connector. Preferably the housing hermetically isolates the lens system, sensor, and components of the driver from the surrounding environment. The housing is preferably fabricated from a material selected from the group comprising: titanium, stainless steel, and polymers.

The electronic driver typically includes an amplifying component comprised of an N-channel field effect transistor (FET) or an amplifier circuit, wherein the amplifier circuit can be a Maxim operational amplifier that includes several amplifying stages.

The lens system of the invention typically comprises a plurality of lens that together form an image with a field of view of between 60 and 160 degrees; the field of view may be between 60 and 140 degrees.

In preferred embodiments of the invention, the lens system is designed for carrying out a procedure selected from the following group:
(a) a gastroscopy procedure by forming an image with a field of view of 120 to 140 degrees;
(b) an ERCP procedure by forming an image with a field of view of 120 to 140 degrees in the motherscope and by forming an image with a field of view of 100 degrees in the baby scope;
(c) a colonoscopy procedure by forming an image with a field of view of 120 to 140 degrees;
(d) a gynecology procedure by forming an image with a field of view of 100 to 120 degrees;
(e) a bronchoscopy procedure by forming an image with a field of view of 80 to 100 degrees; .
(f) an ENT procedure by forming an image with a field of view of 80 to 100 degrees; and
(g) a transgastric procedure by forming an image with a field of view of 100 to 140 degrees in the motherscope and by forming an image with a field of view of 100 to 120 degrees in the baby scope.

In preferred embodiments of the camera head of the invention, the diagonal size of the solid-state sensor chip is in the range from approximately 1.01mm to approximately 2.84mm and the diameter of the distal tip of the rigid, semi-rigid, or flexible endoscope is in the range from approximately 1.6mm to approximately 3.5mm.

The first lens may be glued to the solid-state sensor.

All the above and other characteristics and advantages of the invention will be further understood through the following illustrative and non-limitative description of preferred embodiments thereof, with reference to the appended drawings in which like parts are designated by the same reference number.

### Brief Description of the Drawings

- Figs. 1 to 3 show a first embodiment of the reusable camera head of the invention;
- Figs. 4A to 4C show an embodiment of the camera head of the invention comprising external electric connectors;
- Figs. 5 to 7 show a reusable camera head not according to the invention;
- Fig. 8 shows the distal end of a disposable endoscope comprising a camera of the invention;
- Fig. 9 and Fig. 10 show the distal end of the endoscope of Fig. 8 with part of the sheath removed;
- Figs. 11 to Fig. 14 show another embodiment the camera head of the invention;
- Fig. 15A, Fig. 15B, and Fig. 15C show respectively examples of configurations of the lens systems having 100 degree, 120 degree and 140 degree fields of view;
- Fig. 16 schematically shows a typical arrangement of a driver for the camera head of the invention that does not comprise a PCB;
- Fig. 17A and Fig. 17B show the electronic components assembly for a camera head comprising a 1/15" CCD chip;
- Fig. 19 shows the 1/15" CCD chip secured in a special jig; for removing the excess tape;
- Fig. 20 shows the CCD chip, legs, and wire terminals after most of the CCD tape has been cut away;
- Fig. 21 shows the CCD chip held in an especially designed holding jig;
- Fig. 22 shows how the wires from the electronic sub-assembly and the two capacitors are attached to the CCD wire terminals by soldering;
- Fig. 23 shows how an electrically insulating pad support is electrically connected to the driver assembly by soldering the pads to the CCD wire terminals;
- Fig. 24A and Fig. 24B show respectively the detachable distal tip of the invention attached to and detached from the distal end of an insertion tube of an endoscope;
- Fig. 25A and Fig. 25B are perspective views showing the detachable distal tip of the invention;
- Fig. 26 is a perspective view showing the distal end of an insertion tube of an endoscope adapted to mate with the detachable distal tip of the invention; and
- Fig. 27 is a cross-sectional view taken along the longitudinal axis of Fig. 24A.

### Detailed Description of Preferred Embodiments

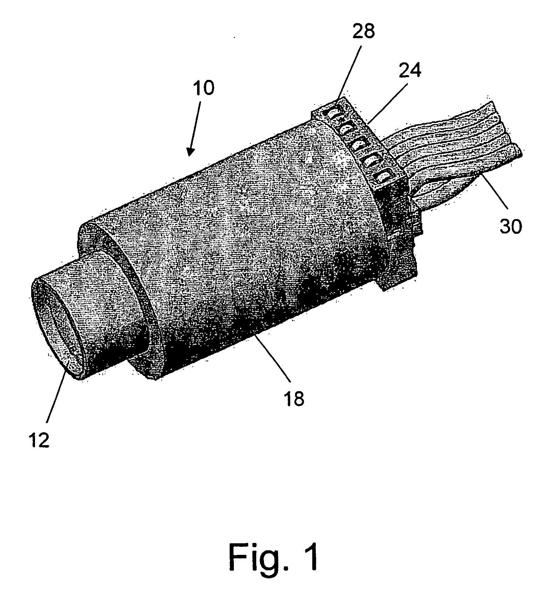

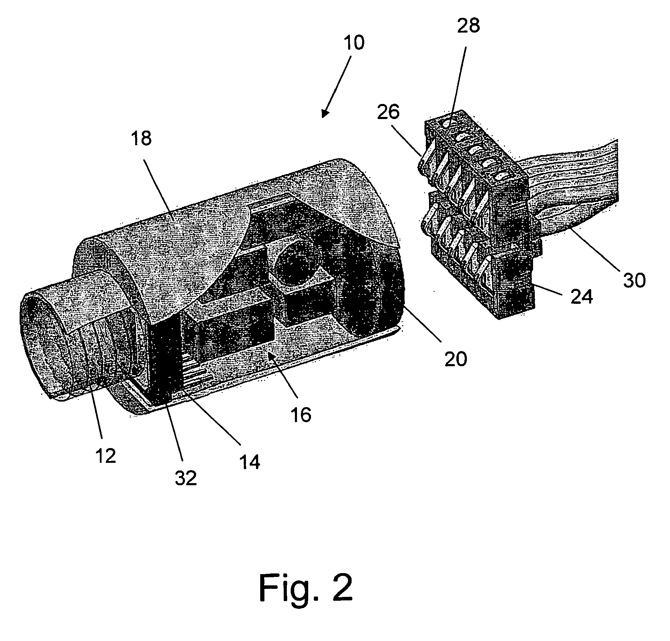

Figs. 1 to 3 show a first embodiment of the reusable camera head 10 of the invention. Referring first to Fig. 1, camera head 10 is a closed housing 18, preferably cylindrically shaped, with a lens system 12 at its front end. The camera head 10 is shown in Fig. 1 attached to external electrical connector 24. External electrical connector 24 has around its outer perimeter clasps 28, which can be for example leaf springs, which are used to hold external electric connector in position in a socket at the distal end of the endoscope. Signal transmission and electric power supply wires 30 are attached to the back face of external electrical connector 24 and are in electrical contact with a plurality of pins 26 (see Fig. 2) arranged in a pattern on the front face of connector 24.

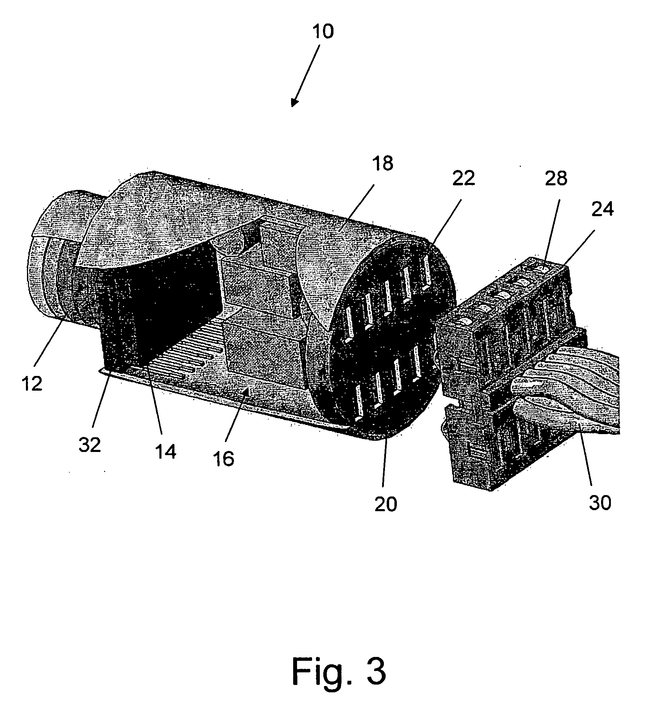

Fig. 2 and Fig.3 show the camera head 10 with part of the housing 18 removed to reveal the interior of the camera head. The camera head 10 is shown disconnected from the external electrical connector 24 in these figures. Generally, in all of the figures herein, the internal electrical connections between components of the camera head are not shown for clarity.

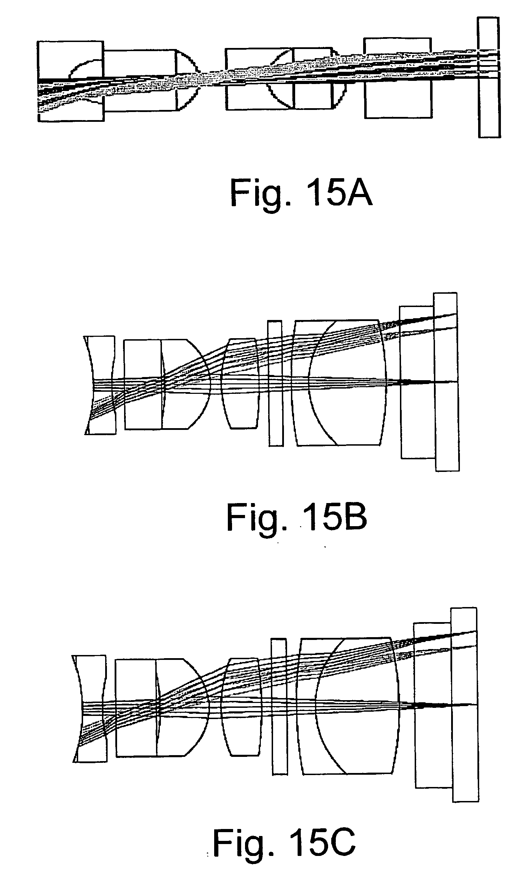

Inside of the housing 18 is a front wall 32 that supports the lens system 12 on its front face and the CCD 14 on its rear face. The lens system 12 is of conventional design for endoscopic or laparoscopic instruments. The lenses are made of material that can withstand repeated sterilization, particularly autoclaving, procedures. Suitable examples are, for example N-SK10 and N-SF8 optical glasses supplied by Schott Glass Technologies or glasses with Tg>530 centigrade. The lens system typically comprises a plurality of lens that together (depending on the combination of lenses used) form an image with a field of view of between 60 and 160 degrees and depth of focus suitable from 1mm to 100mm in front of the camera head and project the acquired image on the face of the CCD. Examples of configurations of the lens systems having 100 degree, 120 degree and 140 degree fields of view are shown in Fig. 15A, Fig. 15B, and Fig. 15C respectively. The field of view of the lens system of the camera is selected according to the type of procedure to be carried out by the endoscope to which the camera will be attached. Some procedures are performed using two endoscopic devices: a "mother scope" which is used to gain access to the region of the procedure within the body and a second, smaller diameter, "baby scope" that is used to carry out the procedure. The field of view of the baby scope is typically smaller than that of the mother scope. Examples of various procedures in the visible radiation band and the corresponding field of view are shown in Table 1.

**Table 1**

| Procedure | Field of view |
|---|---|
| Gastroscopy (EGD) | 120 - 140 |
| ERCP | 120 -140 (mother scope) |
| | 100 (baby scope) |
| Colonoscopy | 120 - 140 |
| Gynecology | 100 - 120 |
| Bronchoscopy | 80 - 100 |
| Transgastric | 100 - 140 (mother scope) |
| | 120-100 (baby scope) |
| ENT | 80 - 100 |

Similar tables can be constructed for procedures carried out while observing in bands other than the visible, for example, for angiography where the CCD would be sensitive in the x-ray band.

Examples of commercially available CCD chips 14 for use in camera head 10 are ICX256/7FKW CCD (1/10" diagonal) manufactured by Sony, and LC99267FSB CCD (1/9") by Sanyo. Sony has also developed a 1/15" CCD sensor (ICX421FKZ). Sensors having CCD chips with even smaller diagonal dimensions are presently being designed. In preferred embodiments of the invention, the lens system 12 is attached to the face of the CCD 14 using a heat resistant optically transparent adhesive that has a thermal expansion coefficient that will prevent damage to the camera head during sterilization procedures carried out in an autoclave.

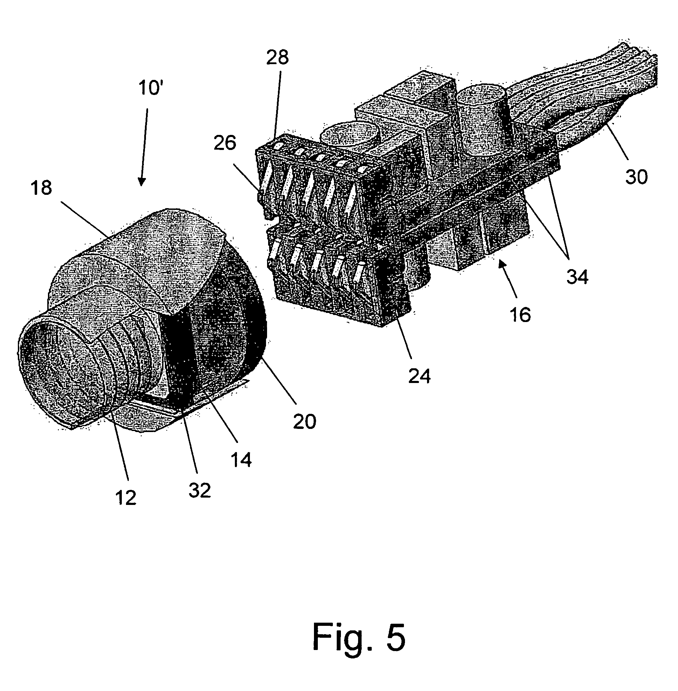

Also shown inside the housing of camera head 10 are electronic components of electronic driver 16. For the larger sized CCD chips, as is done in prior art camera heads, some all of the components of driver 16 can be mounted in a fashion not according to the invention on one or more ceramic or polymer, for example Teflon, printed circuit boards (PCBs) 34 (shown in Fig. 5, but not seen in Fig. 2 or Fig.3). Circuitry is printed on the surface of the PCB and the electronic components, which include an amplifying component, are attached. An N-channel field effect transistor (FET) is usually used as an amplifying component in driver designs. An amplifier circuit such as a Maxim operational amplifier that includes several amplifying stages may implement the amplifying element. In order to reduce the physical dimensions of driver 16, the resistive components may be implemented by utilizing burn resistors. The burn-resistors are created in the PCB conducting lines, and serve as electrical links between the electrical components. In this way the space consumed by the resistive components becomes negligible.

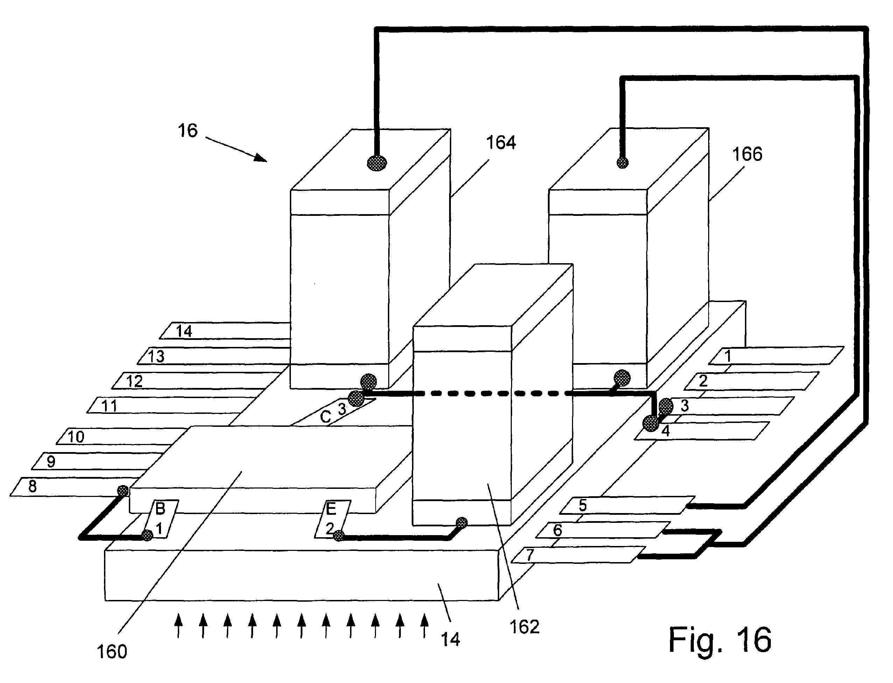

In the preferred embodiment of the detachable camera head, especially for those comprising the smallest sized CCD chips, no PCB is used in the camera head. The components of the driver are attached directly to the back surface of the CCD chip, to the connector, or to the inside walls of the housing. A typical arrangement of driver 16 for the camera head of the invention that does not use a PCB is schematically shown in Fig. 16. On the back of the CCD chip 14 (1/10" - ICX257FKW) are glued transistor 160 (2S2029 ROHM), resistor 162 (51Ohm), capacitor 164 (1nF, 50V), and capacitor 166 (100nF, 16V). The internal connections are shown in Fig. 16. The fourteen tabs on the CCD chip and the top electrode of the resistor are connected to the internal electrical connector 20 (see Fig. 2). It is stressed that the arrangement shown in Fig. 16, and especially the component numbers and parameters given in parenthesis, are given merely to illustrate one way of implementing driver 16 and is not meant to limit the invention in any way. An example of another method of assembly of a miniature camera comprising a 1/10" CCD chip will be described hereinbelow.

Referring again to Fig. 2 and Fig. 3, all signal transmission lines and electrical supply wires to and from the driver 16 and CCD 14 are connected to contacts on the inside face of internal electric connector 20. The contacts on the inner face of internal electric connector 20 are in electrical contact with a plurality of sockets 22 arranged in a pattern on the outside face of connector 20. The pins 26 on the front face of external electric connector 24 fit into the sockets 22 on the outside face of internal electric connector 20, thus establishing electrical continuity between the components inside housing 18 of reusable camera head 10 and signal transmission and electric power supply wires 30 in the endoscope, the distal ends of which are electrically connected to the pins in external electric connector 24. In addition, since the external electrical connector is fixedly attached to the endoscope by means of clasps 28, the pins 26 and sockets 22 act as a "quick connector" for attaching the camera head 10 to the endoscope.

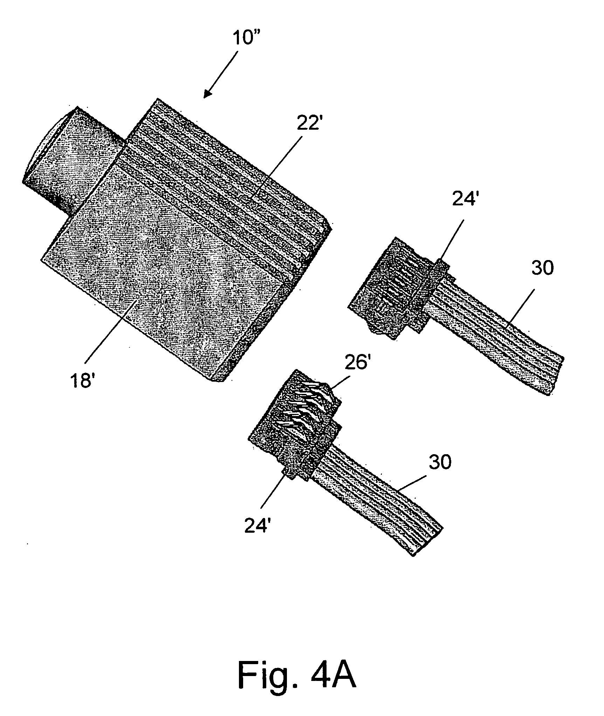

For the electrical connectors shown in Fig. 2 and Fig. 3, the pins and sockets are arranged internally on the faces of the respective connectors. In Figs. 4A to 4C is shown another arrangement for making the electrical contact between the interior of the camera head and its exterior. As seen in Fig. 4A, the housing 18' of camera head 10" has a rectilinear shape. Housing 18' is made of an electrically insulating material and in the planar upper and lower surfaces are longitudinal slots containing metal electricity conducting strips 22' that are electrically connected to the inside. The external electrical conductor is in two parts 24' that are fixedly attached to the walls of a socket in the distal end of the endoscope and spaced apart such that camera head 10" can be inserted between them. On the face of each part 24' of the external electric conductor are metallic contacts 26' that are arranged in a matching pattern to the conducting strips 22' on the top and bottom surfaces of housing 18' and to the signal transmission and electric power supply wires 30. Metallic contacts 26' are preferably leaf springs that will provide electrical contact and also will act as a "quick connector" holding the camera head at the distal tip when it has been pushed into place and allowing it to be detached easily simply by pulling it away from the endoscope.

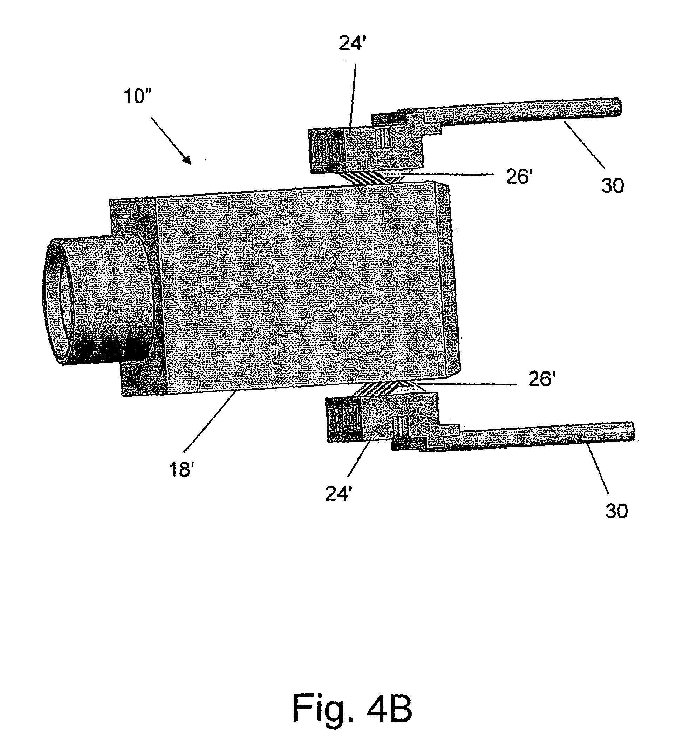

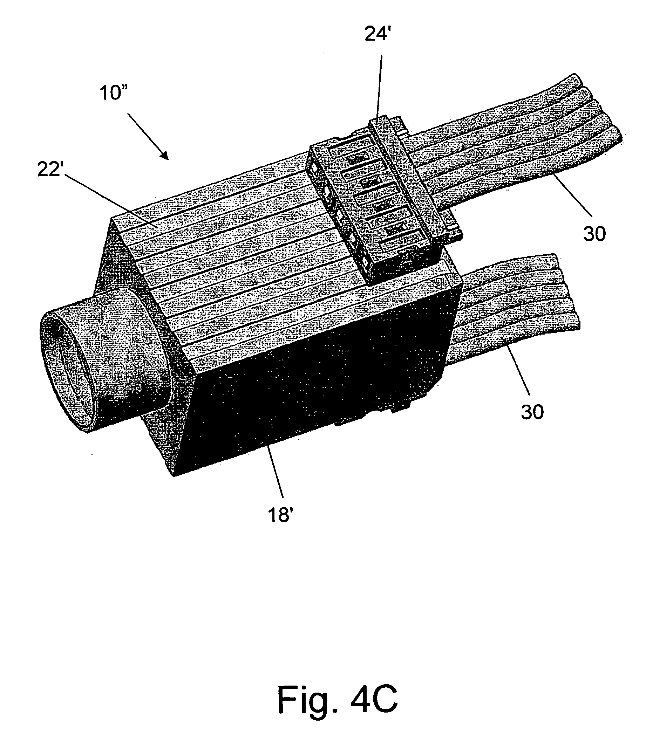

Figs. 4B and 4C show Camera head 10' held in place between the two parts 24' of the external electric connector. The arrangement shown in Figs. 4A to 4C can be utilized to reduce the lateral dimensions of the camera head under certain conditions.

The housing 18 completely surrounds the components of camera head 10, thereby hermetically isolating the components of the camera head, except for the front surface of the first lens in the optical system and the outer face of the electrical connector 22, from contact with the surrounding environment. Thus, as long as all the components of the camera head are heat resistant up to the temperatures reached in an autoclave, no special precautions have to be taken when using the camera or during the sterilization procedure. The housing must be resistant to sterilization solutions such as CIDEX and EtO and autoclave sterilization at 134°C and pressure of 2.3 bar. Suitable materials from which the housing can be fabricated are, for example, titanium, stainless steel, or a polymer, such as Teflon or one of its derivatives.

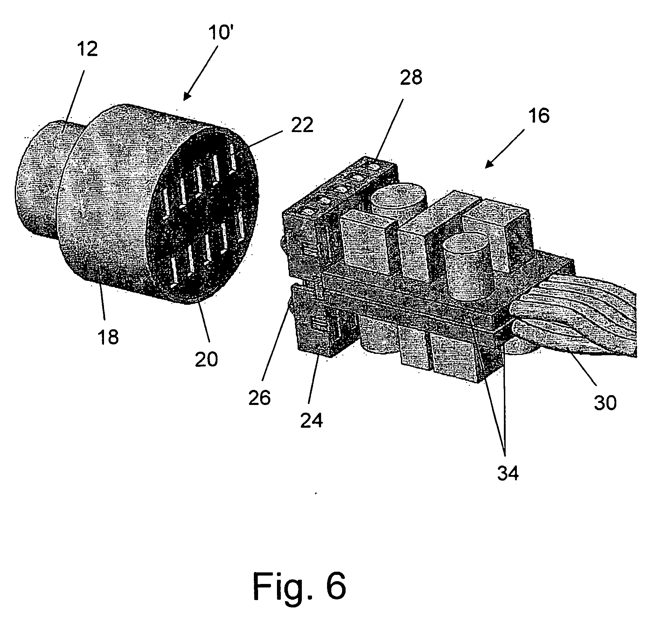

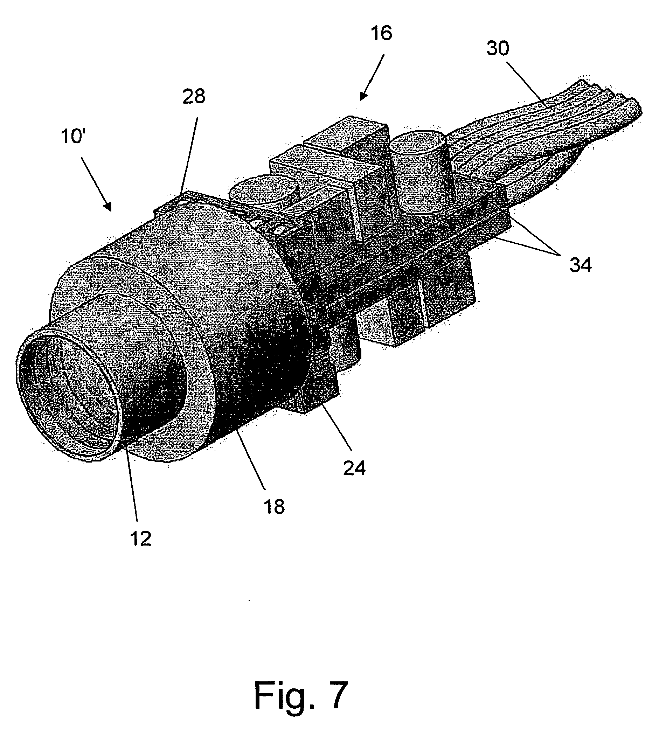

Figs. 5 to 7 show a comparative example not according to the invention of the reusable camera head 10' . Here, electronic driver 16 is not inside of housing 18 of the reusable camera head 10', but is located proximally of the external electrical connector 24. This makes the driver 16, part of the disposable endoscope and therefore the components can be made having less strict tolerances and are therefore less expensive than those of the embodiment described hereinabove. This follows not only because the driver does not have to be able to survive the elevated temperature of autoclave sterilization, but also because the requirements of circuit stability, etc. are much less stringent for a unit that is only required to work once than for a unit which is designed to operate repeatedly over many thousands of cycles. Skilled persons will also realize that the driver 16 can be produced as an ASIC component, greatly reducing the cost of the camera head. Additionally, the cable that is used to connect between the external connector and the video processor in the present invention can be designed to a much lower standard than is possible with prior art cameras.

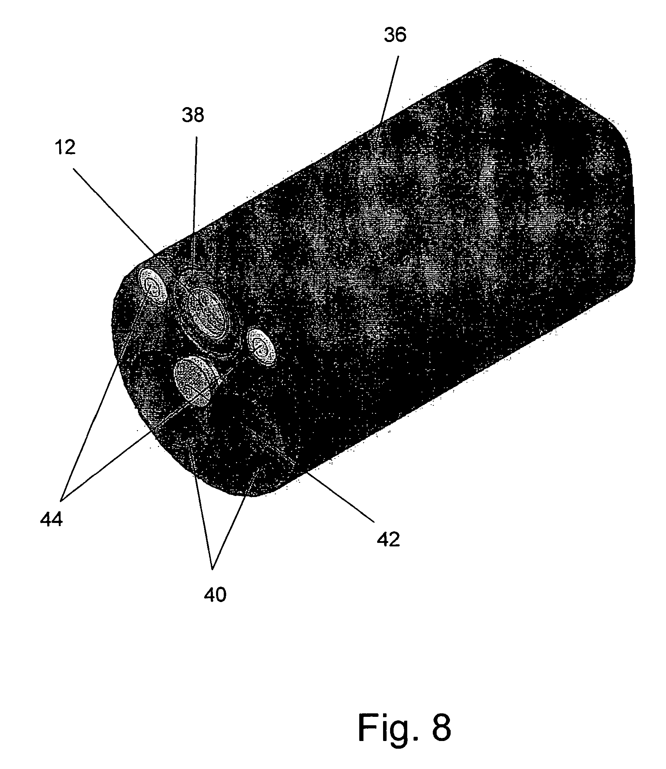

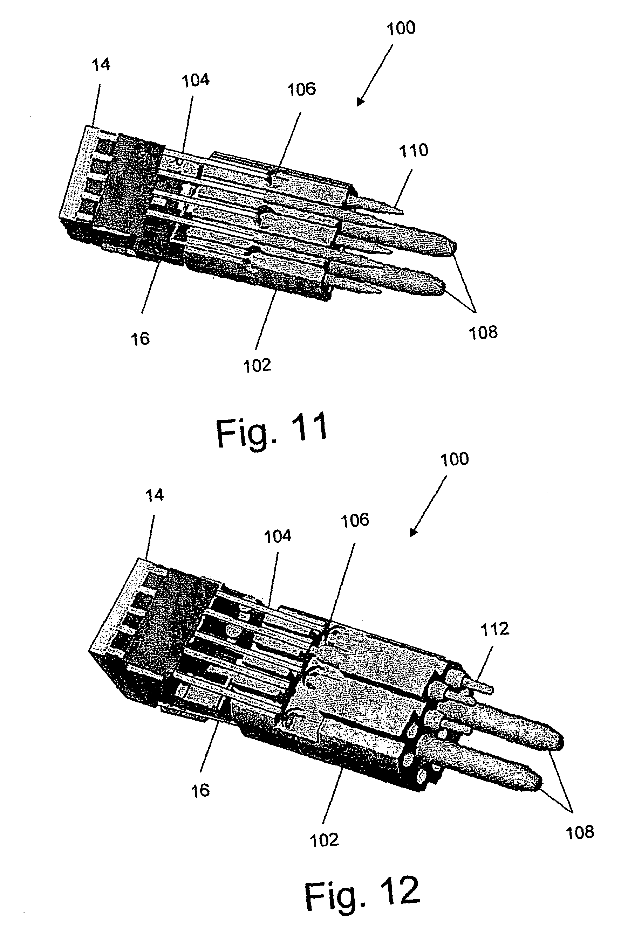

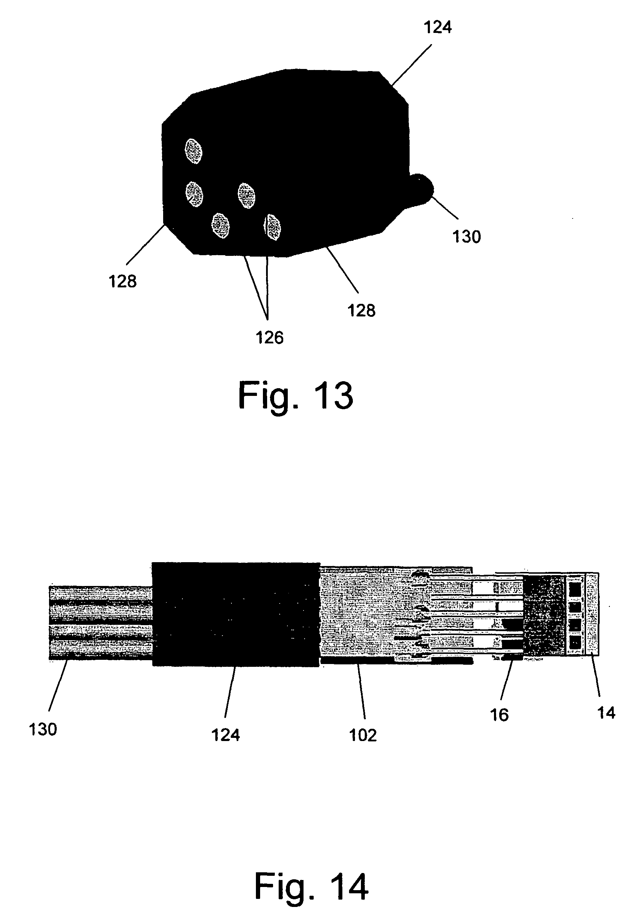

Figures 11 to 14 show another way of implementing the camera head of the invention. In this embodiment of the camera head 100, the internal connector is replaced with a separate insulating conductor support 102. The camera head is shown without the lens system mounted on the CCD 14 and with the housing that surrounds and protects it removed. The components of driver 16 are attached to the CCD 14 or the conductor support 102 without the use of a PCB as described with reference to Fig. 16. In Figs. 11 and 12 the CCD 14 has ten tabs connected to CCD conductors 104, which extend to and are bent into slot 106 in the top of conductor support 102. Projecting out of the proximal face of the conductor support 102 are two guide pins 108 for aligning the conductor support 112 with a matching connector 124 (Fig. 13) and for guiding the ten pins on the conductor support 112 into matching bores on the face of the connector 124. Fig. 11 shows an embodiment in which the ten pins 110 are rigid and slide into a metal sheath lining the inside of the bores on the face of connector 124. In this embodiment the end of each pin is pushed against the bent end of the corresponding CCD conductor 104 in slot 106 and soldered to it. Fig. 12 shows an embodiment in which spring probes 112 are installed in the conductor support 102. In this case, it is not necessary to solder the CCD conductors to the probes, since the force exerted by the compressed springs in the probe 102 when it is inserted into the bore in connector 124 will be sufficient to provide electrical continuity. In Fig. 13 is shown a connector 124 for use with the embodiment shown in Fig. 12. A ten-conductor camera cable 130 is attached to the proximal face of connector 124. On the distal face of connector 124 can be seen ten bores 126 comprising metallic cores against which the ends of the spring probes 112 are compressed as the guiding pins 108 are pushed into the two guiding bores 126. In order to ensure good electrical connection between the contacts on the internal and external connectors, a conducting layer, for example, GB MATRIX TYPE by Shin-Etsu Chemical Co., Ltd. Can be attached between connectors 102 and 124 (shown in Fig. 14), e.g. by attaching it to the distal face of connector 124 (Fig. 13). Fig. 14 is a side view showing the conductor support 102 with its attached CCD assembly connected to camera cable 130 by means of cable connector 124. Fig. 8 shows the distal end of a disposable endoscope 36 comprising a camera head 10,10' of the invention. The choice of a disposable endoscope is merely for illustrative purposes and the camera head of the invention can also be used with any type of endoscope. The endoscope need not be disposable after a single use, but the entire device or some of its components can be sterilized separately from the camera head and reused for subsequent procedures.

Shown on the distal face of endoscope 36 is the surface of the first lens of lens system 12; a gasket 38, which surrounds the lens system to prevent liquids from entering the interior of endoscope; two light fibers 44, which illuminate the area viewed by the camera; an irrigation nozzle 42, to clean the lenses; and two working channels 40, through which the surgeon/gastroenterologist can insert the tools necessary to carry out the procedure. The endoscope shown in Fig. 8 is illustrative only and the reusable camera head of the invention can be attached to rigid, semi-rigid, or flexible endoscopes comprising many different configurations and accessories. As specific examples, the endoscope can comprise only a single working channel or the illumination can be provided by a single fiber, which ends in a light ring around the perimeter of the distal face.

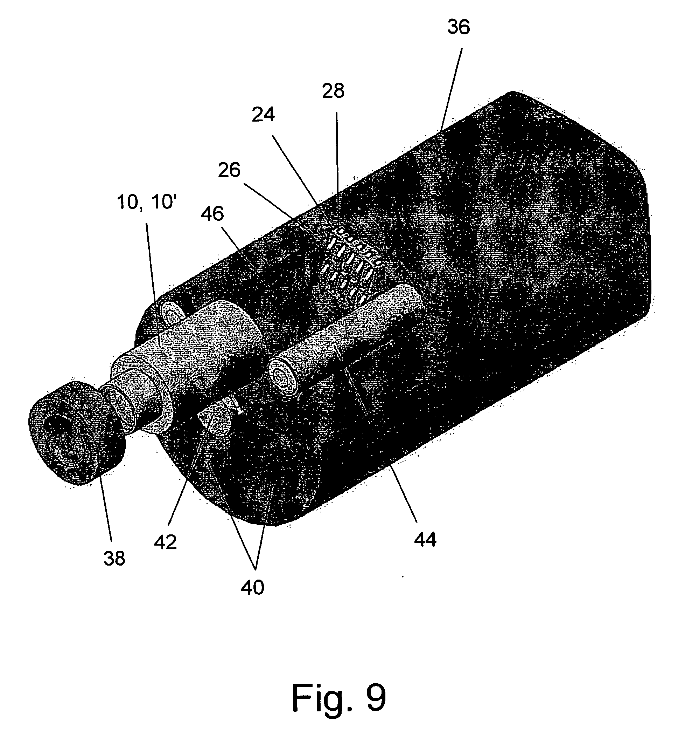

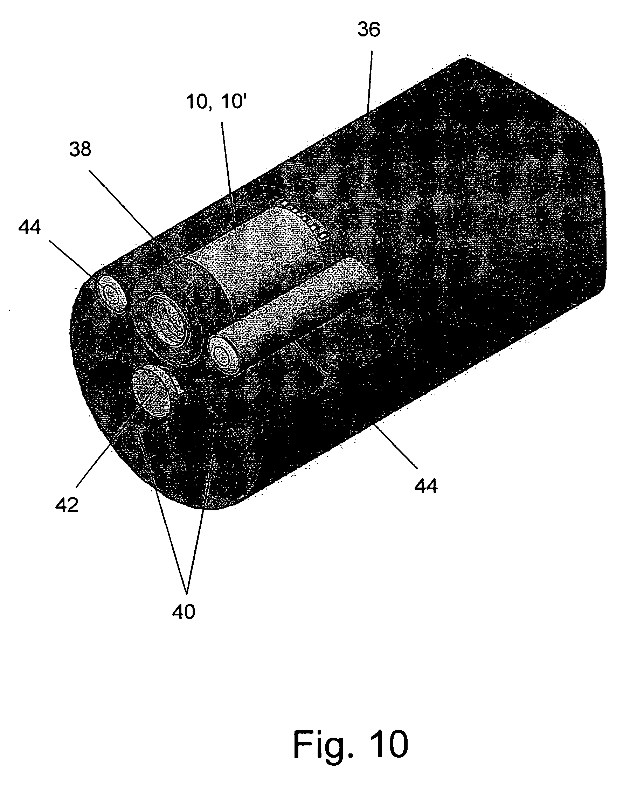

Fig. 9 and Fig. 10 show the distal end of the endoscope 36, with part of the sheath removed. From these two figures, it can be seen how the camera head 10,10' is slid into (and out of) socket 46 in the distal end of endoscope 36 and is pushed into (and out of) the external electrical connector 24 that is attached to the endoscope.

Referring to Figs. 8 to 10, the working channels 40 have a diameter of 0.8-1.2mm, the light fibers 44 a diameter of 0.3-0.6mm, and the diameter of the camera head is 3mm. The external diameter of the endoscope is less than 5mm and if the endoscope has only one working channel it will have an external diameter of up to 4mm. In table 2 are shown dimensions for endoscopes that will use detachable camera heads without a PCB and the CCD's presently available or under development.

**Table 2**

| Tip | Light | Irrigation | Camera head | CCD chip |
|---|---|---|---|---|
| diameter | fibers | channel | Min diagonal size | size |
| (mm) | OD (mm) | (mm) | (mm) | (inches) |
| 3.5 | 1.17 | 0.6 | 2.54 | 1/10 |
| 3.0 | 0.7 | 0.4 | 1.69 | 1/15 |
| 2.3 | 0.6 | 0.3 | 1.41 | 1/18 |

The presently preferred embodiment of the assembly procedure for the miniature camera head of the invention will now be described. The procedure described herein pertains to the 1/10" CCD chip but is provided merely to illustrate the invention and is not intended to limit the scope of the invention in any manner. For example, it is expected that the same procedure will be used *mutatis mutandis* with a 1/15" and smaller CCD chips. The CCD chip has the shape of a square having sides of approximately 1.8mm length. Therefore only a transistor and a resistor are attached directly to the CCD chip and the two capacitors, which comprise the remainder of the electronic driver are added to the assembly at a later stage.

Because of the small size of the components and also to insure the accuracy of the assembly, the components are handled either manually using micro tweezers or a vacuum pick-and-place device or automatically with an automatic pick-and-place machine and the assembly is carried out using a stereomicroscope such as Nikon SMZ 800.

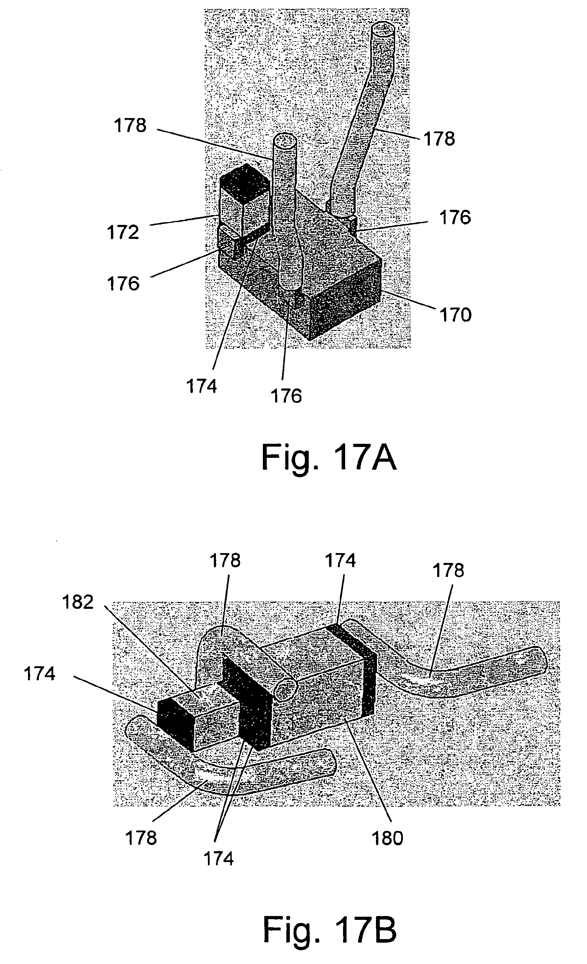

The first stage of the procedure is the assembly of the electronic components. The transistor (e.g. type 2sa2029, pack 0402) is placed on a flat surface using, for example, micro tweezers. Next UV glue, such as Loctite type 3494 is applied on one side of the transistor. To insure accurate application of the glue, a glue dispenser, for example an ESD 1400 glue dispenser, is used. After the glue is cured using a UV light curing system such as Dymax blue wave 50, a resistor (e.g. 39 ohm, pack 0201) is placed on the transistor. Fig. 17A shows the electronic components assembly. In the figure it can be seen how the five-sided termination 174 at one end of resistor 172 is pushed into contact with one of the three conducting legs 176 of transistor 170. After another layer of UV glue is applied and cured, the electronic components are connected to each other and to jumper wires 178 using precision soldering devices, e.g. manual soldering stations like Weller MT 1500 or a high-power diode laser such as one of those produced by Dilas GmbH systems.

Now, using the soldering device, the two capacitors are soldered to each other and to jumper wires as shown in Fig. 17B. Capacitor 180 has a capacitance of 100nF and capacitor 182 has a capacitance of 1nF.

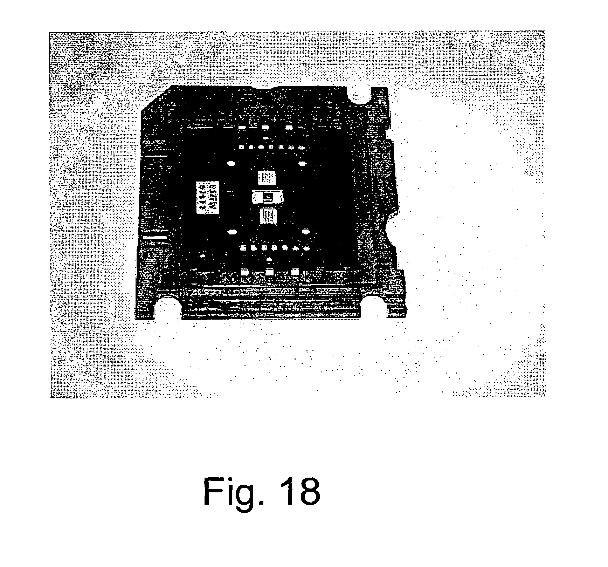

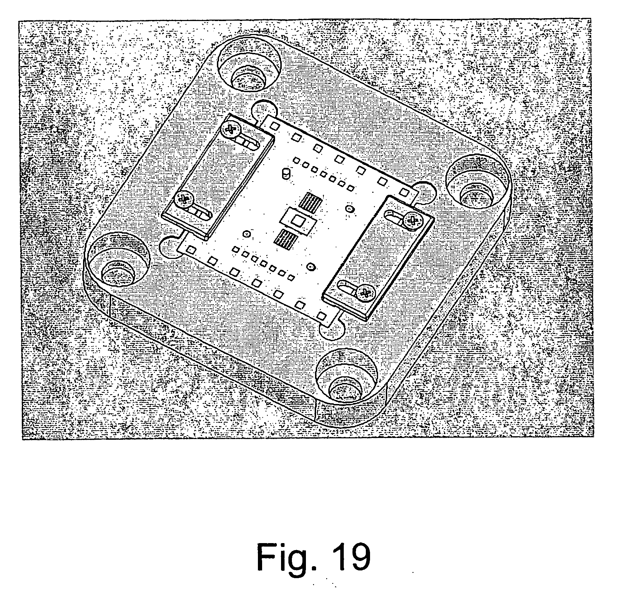

The next stage of the procedure is to prepare the CCD so that the electronic components can be attached to it. Fig. 18 shows the 1/10" CCD chip (Sony ICX257) with a plastic holder surrounding the tape, as it is supplied by the manufacturer. The plastic holder surrounding the tape is removed from the CCD component and the CCD tape is secured in a specially designed jig (shown in Fig. 19). Initially the CCD tape is secured with the photosensitive side of the chip facing upwards and a glass spacer, to which the lens system will eventually be attached, is glued to the CCD's glass side using optical glue such as 140-M light curing glue by DYMAX Corporation.

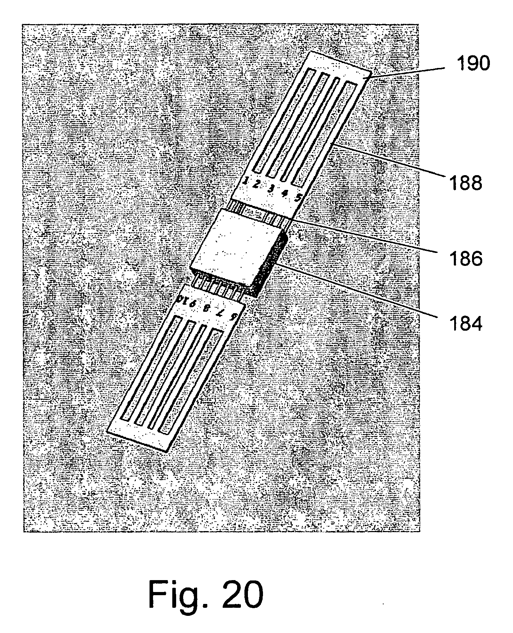

The CCD is now released from the jig, turned over such that the glass is at the bottom, and it is replaced in the special jig. Most of the CCD tape is now cut away using a scalpel. Fig. 20 shows the results of this operation comprising the CCD chip 184 with its legs 186 and CCD wire terminals 188. A small strip of the CCD tape 190 is left to maintain the mechanical integrity of the chip.

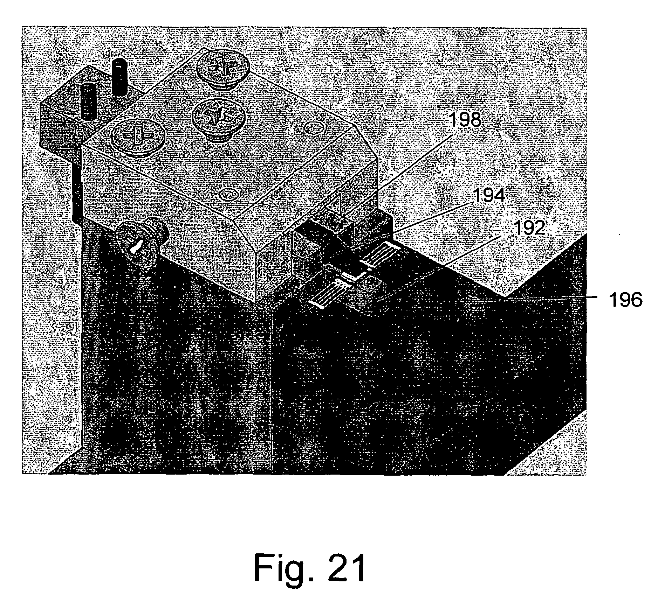

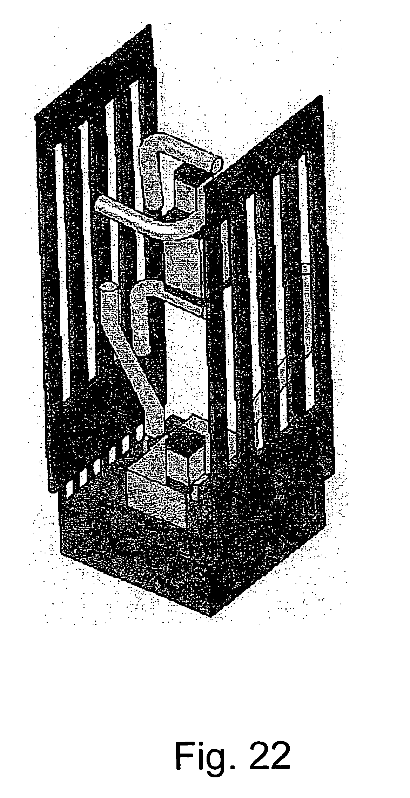

The chip is now transferred to the especially designed holding jig shown in Fig. 21. The chip is centered on chip support 192 and held firmly in place by tongue 194. Z stage 196 is now raised past the chip support 196 causing CCD legs 186 to bend upwards around the sides of tongue 194 until they are bent into the configuration shown in Fig. 22. Referring again to Fig. 21, side walls 198 are advanced out of the interior of the holding jig to support the CCD wire terminals 188. The Z stage is now moved upwards until it releases the CCD legs. The tongue 194 is now retracted into the jig and repositioned 0.3mm from the edge of the CCD chip in order to clamp the chip in place while leaving room to attach the electronic components. The holding jig is now moved to the micromanipulator table where the electronic assembly will be completed.

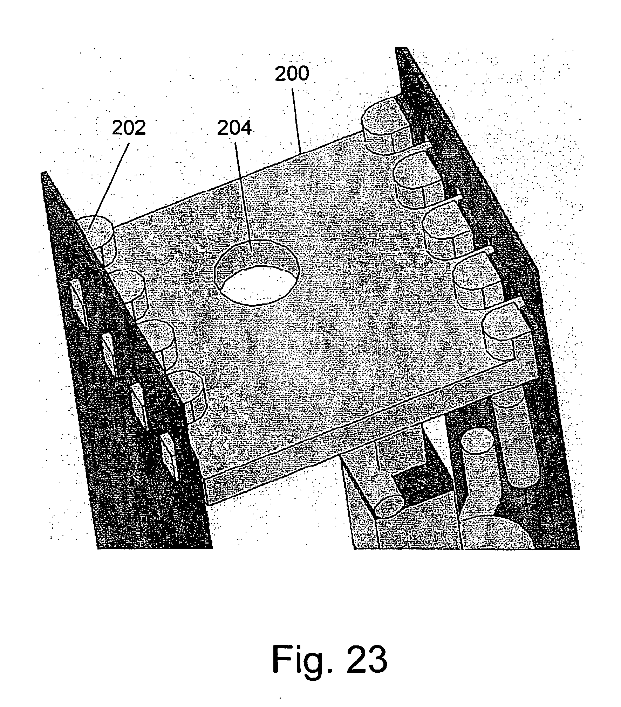

The previously prepared electronic components assembly (Fig. 17A) is placed on the CCD and secured to it by using UV glue such as 204-CTH by DYMAX Corporation using a precise glue dispenser such EFD ULTRA 1400 + micros dispense pen system and UV light curing system. The wires from the electronic sub-assembly and the two capacitors (Fig. 17B) are attached to the CCD wire terminals by soldering as shown in Fig. 22. The next step in the assembly procedure is shown in Fig. 23. An electrically insulating pad support 200 comprising nine electrically conducting pads 202 and a hole 204 is placed between the CCD wire terminals above the capacitors and electrically connected to the driver assembly by soldering the pads to the CCD wire terminals. Finally, one wire is passed through hole 204 and soldered to the resistor and nine more wires are soldered to each of the pads 202. Once the electrical connections have been completed the entire assembly, from the back of the CCD to the top of the pads, is encapsulated using UV glue.

The encapsulated CCD is now turned over and a lens holder comprising a typically cylindrically shaped sleeve and the remainder of the elements of the lens system is glued to the CCD on the side where the glass is located. The encapsulated CCD is now placed into a suitably shaped titanium or stainless steel housing with the lens holder preferably projecting out of the front end of the housing. The ten wires at the top of the assembly are threaded through the titanium housing and the titanium housing is sealed to the lens-housing holder using biocompatible epoxy, for example EPO-TEK 353 ND.

Next, the 10 wires are connected by soldering or crimping to ten male micro connectors in a conductor support. The connectors comprise special pins such as Mill-Max Mfg. Corp cat. no. 8210 or Interconnect Devices, Inc. IDINET penta 0.

Finally the housing is filled with epoxy and closed by placing the connector support in its proximal end. Any excess glue is then wiped off to complete the creation of a totally closed (encapsulated) structure.

The housing of a camera head assembled according to the above described procedure and based on a 1/10' CCD will be on the order of 3-4mm in diameter. From a practical point-of-view handling such a small camera head, for example when removing it from an endoscope, sterilizing it, or reattaching it to the same or another endoscope for another procedure will be an awkward procedure for the practitioner to carry out. To overcome this problem, in a preferred embodiment of the invention, the miniature camera head will be a permanent part of a sterilizable distal tip, which can be detached and reattached to the insertion tube of an endoscopic or laparoscopic device.

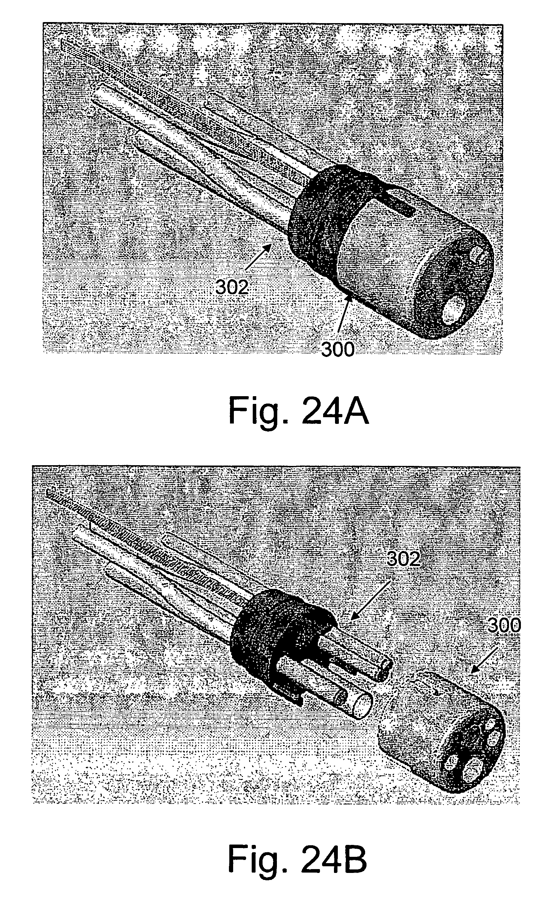

Fig. 24A and Fig. 24B show respectively the detachable distal tip of the invention 300 attached to and detached from the distal end of an insertion tube of an endoscope. In these and the following figures, the sheath surrounding the insertion tube and the articulation section, if the endoscope comprises one, is not shown.

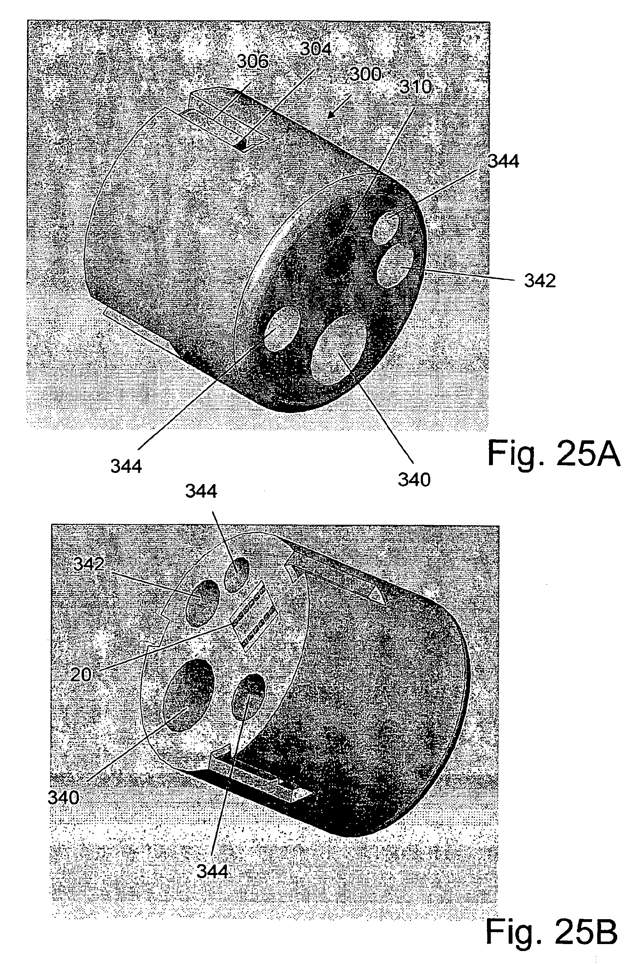

Figs. 25A and 25B are perspective views showing the distal and proximal faces respectively of the detachable distal tip of the invention. Distal tip of the invention 300 is made of a monolithic block of biocompatible material, e.g. a plastic polymer, stainless steel and Titanium. A number of bores through which the various channels of the endoscope pass to the distal tip pass longitudinally through the distal tip. In the figures can be seen bores for the working channel 340, irrigation channel 342, and two light channels 344. A miniature camera head 310 of the type described hereinabove is embedded into distal tip 300 with the front lens of camera flush with the distal end of the distal tip and the internal connector 20 of the camera flush with the proximal end. In the outer surface of proximal end of distal tip 300 are several grooves 306, each having a slot 304 at its distal end that are used for fastening distal tip 300 to insertion tube 302 as will be described hereinbelow.

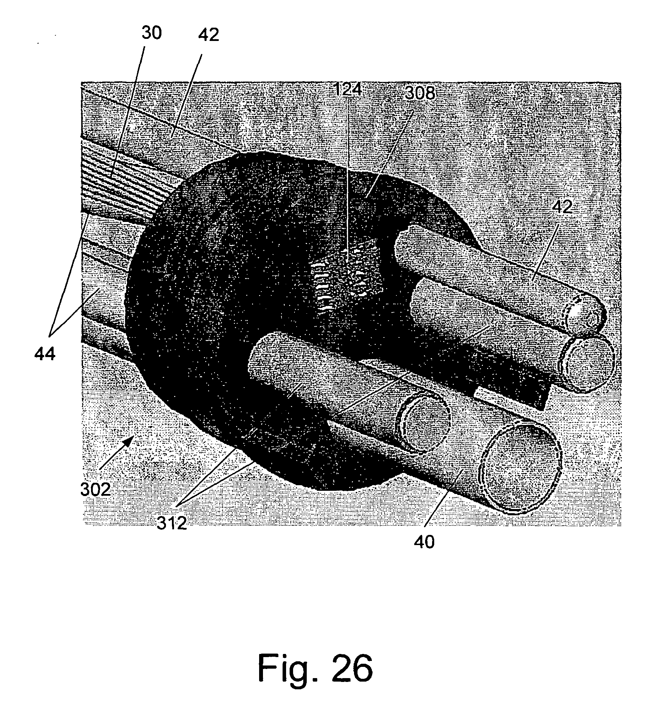

Fig. 26 is a perspective view showing the distal end of an insertion tube of an endoscope 308 adapted to mate with the detachable distal tip of the invention. The interface between the distal end of the insertion tube 302 and the detachable distal tip 300 must be designed such that continuity of the various channels in the insertion tube will be preserved until the distal face. This is accomplished by extending the tubes that define these channels through the insertion tube such that they project out from the distal face. As can be seen in the figures, the irrigation channel 42, working channel 40, and two channels for the optical fibers 312 will slide into matching bores 342, 340, and 344 respectively in the distal tip. As a result when pressed together the extended channels slip tightly into the bores forming a tight seal between the insertion tube as well as providing mechanical strength to the connection between the two parts. To prevent possible damage to the optical fibers when the distal tip is attached or detached, channels 312 can be metal tubes with electro polished interiors or metal tubes filed with Grin lenses to diffuse the light and, if necessary, lenses at the distal end to properly distribute the light in the field of view of the camera. The optical fiber light guides in the insertion tube can end at the interface and light exiting the fibers coupled into the proximal ends of channels 312, thereby providing optical continuity from the light source located proximally of the endoscope to the distal face. It is also possible to use light emitting diodes (LEDs) oor polymer light emitting diodes (PLEDs) located at the distal end of the insertion tube instead of illumination fibers. The internal electrical connector 20 of the camera 30 located on the proximal end of the distal tip 300 is forced into electrical contact with the external electrical connection 124 on the distal end of the insertion tube 302, thereby permitting transfer of electrical power and signals to/from the camera head from/to the peripheral equipment at the proximal end of the endoscope as has been described hereinabove. Because of the very small size of the contacts on the camera head and distal end of the insertion tube, there is a possibility that good electrical connection might not be established; therefore it is preferred to use a matrix type conducting layer at the interface between the contacts as described hereinabove.

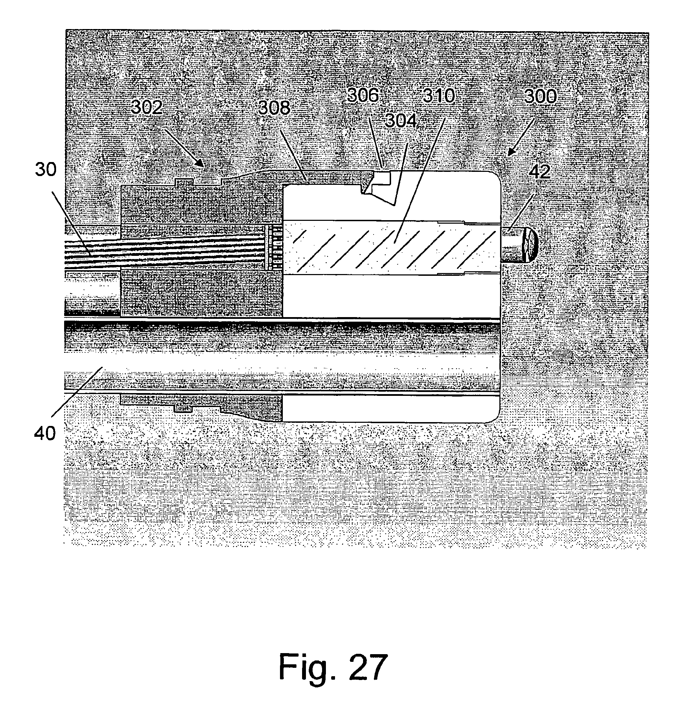

Fig. 27 is a cross-sectional view taken along the longitudinal axis of Fig. 24A. In this figure it can be clearly seen how clasps 308, on the distal end of the insertion tube 302, fit into grooves 806 on distal tip 300 and snap into slots 304 to hold the distal tip in place.

Although embodiments of the invention have been described by way of illustration, it will be understood that the invention may be carried out with many variations, modifications, and adaptations, without departing from or exceeding the scope of the claims. In particular it is to be understood that, although the camera head of the invention has been described herein for use in endoscopy and laparoscopy, it is equally well-suited for use with any other type of probe or device in any conceivable application requiring the use of a miniature camera.

## Claims

1. A reusable miniature camera head (10, 10') that can be attached to and detached from an object (36), said object (36) comprising an external electrical connector (24, 24') fixedly attached to it, said camera head comprising:
- a housing (18, 18');
- a lens system (12) at the distal end of said housing (18, 18');
- a solid-state sensor (14) being a CCD located adjacent to said lens system;
- components (160, 162, 164, 166; 170, 172, 180, 182) of an electronic driver (16); and
- an internal electrical connector (22, 22'; 110; 112) located at the proximal end of said housing (18, 18');
wherein: said camera head (10, 10') is attached to or disconnected from said object (36) by respectively engaging or disengaging electrical contacts on said internal electrical connector (22, 22'; 110; 112) with matching electrical contacts on said external electrical connector (24, 24');
**characterized in that** said camera head (10) does not contain a printed circuit board (PCB), and **in that** said components (160, 162, 164, 166; 170, 172, 180, 182) of said electronic driver (16) comprise discrete components at least one of which is a transistor (160; 170), wherein at least said transistor (160; 170) is glued directly to the back of said solid state sensor (14).

2. A camera head according to claim 1, wherein the solid-state sensor (14) is selected from the following group:
- a Change Coupled Device (CCD);
- an Intensified Charge Coupled Device (ICCD);
- an Electron Multiplying Charge Coupled Device (EMCCD); and
- a Complementary Metal Oxide Semiconductor (CMOS) device.

3. A camera head according to claim 1, wherein at least some of the components of the electronic driver are located outside of the housing.

4. A camera head according to claim 1, wherein the object is a rigid, semi-rigid, or flexible endoscope (36).

5. A camera head according to claim 1, wherein said camera head (10, 10') can be sterilized.

6. A camera head according to claim 5, wherein the sterilization is carried out in an autoclave.

7. A camera head according to claim 5, wherein the sterilization is carried out using chemicals.

8. A camera head according to claim 1, wherein said camera head (10, 10') is a permanent part of a sterilizable distal tip (300) , which can be attached and detached to the distal end of the insertion tube (302) of an endoscopic or laparoscopic device.

9. A camera head according to claim 1, wherein the housing (10') has metallic electricity conducting strips (22') arranged on its outside surface and the external electrical connector (24') has metallic contacts arranged in such a way that they will engage said conducting strips when said housing is pushed into contact with said electrical connector.

10. A camera head according to claim 1, wherein the housing (10, 10') hermetically isolates the lens system (12), sensor (14), and components of the driver from the surrounding environment.

11. A camera head according to claim 1, wherein the housing is fabricated from a material selected from the group comprising:
- titanium;
- stainless steel; and
- polymers.

12. A camera head according to claim 1, wherein the electronic driver includes an amplifying component selected from:
- an N-channel field effect transistor (FET); or
- an amplifier circuit.

13. A camera head according to claim 12, wherein the amplifier circuit is a Maxim operational amplifier that includes several amplifying stages.

14. A camera head according to claim 1, wherein the lens system comprises a plurality of lens that together form an image with a field of view of between 60 and 140 degrees.

15. A camera head according to claim 14, wherein the lens system is designed for carrying out a procedure selected from the following group:
(a) a gastroscopy procedure by forming an image with a field of view of 120 to 140 degrees;
(b) an ERCP procedure by forming an image with a field of view of the camera head of the invention 120 to 140 degrees in the motherscope and by forming an image with a field of view of 100 degrees in the baby scope;
(c) a colonoscopy procedure by forming an image with a field of view of 120 to 140 degrees;
(d) a gynecology procedure by forming an image with a field of view of 100 to 120 degrees;
(e) a bronchoscopy procedure by forming an image with a field of view of 80 to 100 degrees;
(f) an ENT procedure by forming an image with a field of view of 80 to 100 degrees; and
(g) a transgastric procedure by forming an image with a field of view of 100 to 140 degrees in the motherscope and by forming an image with a field of view of 100 to 120 degrees in the baby scope.

16. A camera head according to claim 1, wherein the diagonal size of the solid-state sensor chip is in the range from approximately 1.01mm to approximately 2.54mm.

17. A camera head according to claim 16, wherein the diameter of the distal tip of the endoscope is in the range from approximately 1.6mm to approximately 3.5mm.

18. A camera head according to claim 14, wherein the first lens is glued to the solid-state sensor.

## Patentansprüche

1. Wiederverwendbarer Miniaturkamerakopf (10, 10'), welcher an einem Gegenstand (36) angebracht oder von diesem abgenommen werden kann, wobei der Gegenstand (36) einen an ihm fest angebrachten externen elektrischen Anschluss (24, 24') umfasst, wobei der Kamerakopf umfasst:
- ein Gehäuse (18, 18');
- ein Linsensystem (12) am distalen Ende des Gehäuses (18, 18');
- einen als CCD ausgebildeten Festkörpersensor (14), der benachbart zum Linsensystem angeordnet ist;
- Komponenten (160, 162, 164, 166; 170, 172, 180, 182) eines elektronischen Treibers (16); und
- einen internen elektrischen Anschluss (22, 22'; 110; 112), welcher am proximalen Ende des Gehäuses (18, 18') angeordnet ist;
wobei der Kamerakopf (10, 10') am Gegenstand (36) angebracht wird oder von diesem gelöst wird durch jeweiliges in Eingriff bringen oder außer Eingriff bringen von elektrischen Kontakten am internen elektrischen Anschluss (22, 22'; 110; 112) mit passenden elektrischen Kontakten am externen elektrischen Anschluss (24, 24');
**dadurch gekennzeichnet, dass** der Kamerakopf (10) keine Platine (PCB) enthält und dass die Komponenten (160, 162, 164 166; 170, 172, 180, 182) des elektronischen Treibers (16) diskrete Komponenten umfassen, von denen wenigstens eine ein Transistor (160; 170) ist, wobei wenigstens der Transistor (160; 170) direkt auf die Rückseite des Festkörpersensors (14) geklebt ist.

2. Kamerakopf nach Anspruch 1, wobei der Festkörpersensor (14) aus der folgenden Gruppe ausgewählt ist:
- ladungsgekoppelter Speicher (CCD)
- verdichteter ladungsgekoppelter Speicher (ICCD)
- Elektronen multiplizierender ladungsgekoppelter Speicher (EMCCD); und
- komplementäre Metall-Oxid-Halbleiter-Vorrichtung (CMOS).

3. Kamerakopf nach Anspruch 1, wobei wenigstens einige der Komponenten des elektronischen Treibers außerhalb des Gehäuses angeordnet sind.

4. Kamerakopf nach Anspruch 1, wobei der Gegenstand ein steifes, halb-steifes oder flexibles Endoskop (36) ist.

5. Kamerakopf nach Anspruch 1, wobei der Kamerakopf (10, 10') sterilisiert werden kann.

6. Kamerakopf nach Anspruch 5, wobei die Sterilisation in einem Autoklav durchgeführt wird.

7. Kamerakopf nach Anspruch 5, wobei die Sterilisation unter Verwendung von Chemikalien durchgeführt wird.

8. Kamerakopf nach Anspruch 1, wobei der Kamerakopf (10, 10') ein permanenter Teil einer sterilisierbaren distalen Spitze (300) ist, welche am distalen Ende des Einführungsrohrs (302) einer endoskopischen oder laparoskopischen Vorrichtung angebracht werden kann oder von diesem abgenommen werden kann.

9. Kamerakopf nach Anspruch 1, wobei das Gehäuse (10') metallische, elektrisch leitende Streifen (22') aufweist, die an dessen Außenfläche angeordnet sind, und wobei der externe elektrische Anschluss (24') metallische Kontakte aufweist, die derart angeordnet sind, dass sie mit den leitenden Streifen in Eingriff kommen, wenn das Gehäuse in Kontakt mit dem elektrischen Anschluss gedrückt wird.

10. Kamerakopf nach Anspruch 1, wobei das Gehäuse (10, 10') das Linsenssystem (12), den Sensor (14) und Komponenten des Treibers hermetisch von der Umgebung abdichtet.

11. Kamerakopf nach Anspruch 1, wobei das Gehäuse aus einem Material hergestellt ist, das aus der folgenden Gruppe ausgewählt ist:
- Titan;
- rostfreier Stahl; und
- Polymere.

12. Kamerakopf nach Anspruch 1, wobei der elektronische Treiber eine Verstärkerkomponente umfasst, die aus der folgenden Gruppe ausgewählt ist:
- ein N-Kanal Feldeffekttransistor (FET); oder
- eine Verstärkerschaltung.

13. Kamerakopf nach Anspruch 12, wobei die Verstärkerschaltung ein Maxim Funktionsverstärker ist, der mehrere Verstärkerstufen enthält.

14. Kamerakopf nach Anspruch 1, wobei das Linsensystem mehrere Linsen umfasst, die gemeinsam eine Darstellung bilden mit einem Sichtfeld von zwischen 60 und 140 Grad.

15. Kamerakopf nach Anspruch 14, wobei das Linsensystem dafür ausgebildet ist, ein Verfahren aus der folgenden Gruppe auszuführen:
(a) Gastroskopieverfahren durch bilden einer Darstellung mit einem Sichtfeld von 120 bis 140 Grad;
(b) ein ERCP-Verfahren durch Bilden einer Darstellung mit einem Sichtfeld des Kamerakopfs der Erfindung von 120 bis 140 Grad beim Motherskop und durch Bilden einer Darstellung mit einem Sichtfeld von 10β0 Grad beim Babyskop;
(c) Kolonoskopieverfahren durch Bilden einer Darstellung mit einem Sichtfeld von 120 bis 140 Grad;
(d) Gynäkologisches Verfahren durch Bilden einer Darstellung mit einem Sichtfeld von 100 bis 120 Grad;
(e) Bronchoskopieverfahren durch Bilden einer Darstellung mit einem Sichtfeld von 80 bis 100 Grad;
(f) ein ENT-Verfahren durch Bilden einer Darstellung mit einem Sichtfeld von 80 bis 100 Grad; und
(g) transgastrisches Verfahren durch Bilden einer Darstellung mit einem Sichtfeld von 100 bis 140 Grad beim Motherskop und durch Bilden einer Darstellung mit einem Sichtfeld von 100 bis 120 Grad beim Babyskop.

16. Kamerakopf nach Anspruch 1, wobei die diagonale Größe des Festkörpersensorchip im Bereich von etwa 1,01 mm bis etwa 2,54 mm liegt.

17. Kamerakopf nach Anspruch 16, wobei der Durchmesser der distalen Spitze des Endoskops im Bereich von etwa 1,6 mm bis etwa 3,5 mm liegt.

18. Kamerakopf nach Anspruch 14, wobei die erste Linse auf den Festkörpersensor geklebt ist.

## Revendications

1. Tête de caméra miniature réutilisable (10, 10') qui peut être fixée et retirée d'un objet (36), ledit objet (36) comprenant un connecteur électrique externe (24, 24') fixé fermement à celui-ci, ladite tête de caméra comprenant :
- un boîtier (18, 18'),
- un système d'objectif (12) à l'extrémité distale dudit boîtier (18, 18'),
- un capteur à solide (14) qui est un composant CCD situé de manière contiguë au dit système d'objectif,
- des composants (160, 162, 164, 166 ; 170, 172, 180, 182) d'un circuit électronique d'attaque (16), et
- un connecteur électrique interne (22, 22' ; 110 ; 112) situé à l'extrémité proximale dudit boîtier (18, 18'),
dans laquelle : ladite tête de caméra (10, 10') est fixée ou déconnectée dudit objet (36) en mettant en contact, respectivement en dissociant les contacts électriques se trouvant sur ledit connecteur électrique interne (22, 22' ; 110 ; 112) avec les contacts électriques conjugués sur ledit connecteur électrique externe (24, 24'),
**caractérisée en ce que** ladite tête de caméra (10) ne contient pas de carte à circuit imprimé (PCB) et **en ce que** lesdits composants (160, 162, 164, 166 ; 170, 172, 180, 182) dudit circuit électronique d'attaque (16) comprennent des composants discrets dont au moins un est un transistor (160 ; 170), dans lequel au moins ledit transistor (160 ; 170) est collé directement sur le dos dudit capteur à solide (14).

2. Tête de caméra selon la revendication 1, dans laquelle le capteur à solide (14) est sélectionné à partir du groupe qui suit :
- un dispositif à couplage de charges (CCD)
- un dispositif à couplage de charges intensifié (ICCD)
- un dispositif à couplage de charges à multiplication d'électrons (EMCCD)
- un composant semiconducteur à oxyde de métal, complémentaire (CMOS)

3. Tête de caméra selon la revendication 1, dans laquelle au moins certains des composants du circuit électronique d'attaque sont situés à l'extérieur du boîtier.

4. Tête de caméra selon la revendication 1, dans laquelle l'objet est un endoscope (36) rigide, semi rigide ou souple.

5. Tête de caméra selon la revendication 1, dans laquelle ladite tête de caméra (10, 10') peut être stérilisée.

6. Tête de caméra selon la revendication 5, dans laquelle la stérilisation est réalisée dans un autoclave.

7. Tête de caméra selon la revendication 5, dans laquelle la stérilisation est réalisée en utilisant des produits chimiques.

8. Tête de caméra selon la revendication 1, dans laquelle ladite tête de caméra (10, 10') est une pièce permanente d'une pointe distale (300) pouvant être stérilisée, qui peut être fixée sur l'extrémité distale du tube d'insertion (302) d'un dispositif endoscopique ou laparoscopique et retirée de celle-ci.

9. Tête de caméra selon la revendication 1, dans laquelle le boîtier (10') possède des bandes métalliques (22') conduisant l'électricité, agencées sur sa surface extérieure et dans laquelle le connecteur électrique externe (24') possède des contacts métalliques agencés de manière telle qu'ils coopèrent avec lesdites bandes conductrices lorsque ledit boîtier est mis en contact par poussée avec ledit connecteur électrique.

10. Tête de caméra selon la revendication 1, dans laquelle le boîtier (10, 10') isole hermétiquement le système d'objectif (12), le capteur (14) et les composants du circuit d'attaque par rapport à l'environnement alentour.

11. Tête de caméra selon la revendication 1, dans laquelle le boîtier est fabriqué à partir d'un matériau sélectionné à partir du groupe comprenant :
- le titane,
- l'acier inoxydable, et
- les polymères.

12. Tête de caméra selon la revendication 1, dans laquelle le circuit électronique d'attaque inclut un composant d'amplification sélectionné à partir :
- d'un transistor à effet de champ à canal N, ou
- d'un circuit amplificateur.

13. Tête de caméra selon la revendication 12, dans laquelle le circuit amplificateur est un amplificateur opérationnel Maxim qui inclut plusieurs étages d'amplification.

14. Tête de caméra selon la revendication 1, dans laquelle le système d'objectif comprend une pluralité de lentilles qui forment ensemble une image présentant un champ de vision compris entre 60 et 140 degrés.

15. Tête de caméra selon la revendication 4, dans laquelle le système d'objectif est conçu pour exécuter une procédure sélectionnée à partir du groupe qui suit :
(a) une procédure de gastroscopie en formant une image présentant un champ de vision de 120 à 140 degrés,
(b) une procédure ERCP en formant une image présentant un champ de vision de la tête de caméra de l'invention de 120 à 140 degrés dans le « motherscope » et en formant une image présentant un champ de vision de 100 degrés dans le « baby scope »,
(c) une procédure de colonoscopie en formant une image présentant un champ de vision de 120 à 140 degrés,
(d) une procédure de gynécologie en formant une image présentant un champ de vision de 100 à 120 degrés,
(e) une procédure de bronchoscopie en formant une image présentant un champ de vision de 80 à 100 degrés,
(f) une procédure ENT en formant une image présentant un champ de vision de 80 à 100 degrés, et
(g) une procédure transgastrique en formant une image présentant un champ de vision de 100 à 140 degrés dans le « motherscope » et en formant une image présentant un champ de vision de 100 à 120 degrés dans le « baby scope ».

16. Tête de caméra selon la revendication 1, dans laquelle la taille en diagonale de la puce du capteur à solide se trouve dans la plage allant d'approximativement 1,01 mm à approximativement 2,54 mm.

17. Tête de caméra selon la revendication 6, dans laquelle le diamètre de la pointe distale de l'endoscope se trouve dans la plage allant approximativement de 1,6 mm à approximativement 3,5 mm.

18. Tête de caméra selon la revendication 4, dans laquelle la première lentille est collée sur le capteur à solide.
